(19) Europäisches Patentamt
European Patent Office
Office européen des brevets

(11) Publication number: **0 445 595 A2**

# (12) EUROPEAN PATENT APPLICATION

(21) Application number: **91102540.1**

(22) Date of filing: **21.02.91**

(51) Int. Cl.[5]: **C07C 17/12**, C07C 25/18, C07C 41/22, C07C 43/29, C08L 55/02, C08K 5/03

(30) Priority: **05.03.90 US 487879**
**05.03.90 US 487878**

(43) Date of publication of application:
**11.09.91 Bulletin 91/37**

(84) Designated Contracting States:
**BE DE ES FR GB IT NL**

(71) Applicant: **ETHYL CORPORATION**
**Ethyl Tower 451 Florida Boulevard**
**Baton Rouge Louisiana 70801(US)**

(72) Inventor: **Brackenridge, David Ross**
**5232 Halls Ferry**
**Baton Rouge, Louisiana 70817(US)**
Inventor: **Hussain, Saadat**
**5321 Highland Ridge Drive**
**Baton Rouge, Louisiana 70817(US)**

(74) Representative: **Sandmair, Kurt, Dr. et al**
**Patentanwälte Schwabe, Sandmair, Marx**
**Stuntzstrasse 16**
**W-8000 München 80(DE)**

## (54) Bromination process.

(57) A process is disclosed for preparing a mixture of brominated, non-condensed ring polyaromatics, which process comprises brominating the precursor non-condensed ring polyaromatic in the presence of an $AlCl_3$ catalyst. The mixture has an average bromine number of 6.7 to 7.3 and a hexabromo homolog amount greater than or equal to the sum of the heptabromo and octabromo amounts in the mixture, and it is particularly useful in imparting flame retardancy to ABS formulations and articles made therefrom. The brominated diphenylethane mixtures having an average bromine number of 6.8-7.2 are especially useful in this regard.

EP 0 445 595 A2

This invention relates to a process for producing a novel mixture of brominated non-condensed ring polyaromatics, which mixture has an average bromine number within the range of from about 6.7 to about 7.3.

Mixtures of brominated non-condensed ring polyaromatics are known. For example, brominated diphenyl oxide mixtures having an average bromine number from about 7.0 to about 7.7 are sold commercially as flame retardants for use in thermoplastic formulations. These mixtures conventionally contain 0-2 weight percent hexabromodiphenyl oxide, 40-55 weight percent heptabromodiphenyl oxide, 30-40 weight percent octabromodiphenyl oxide, 5-15 weight percent nonabromodiphenyl oxide and 0-2 weight percent decabromodiphenyl oxide. Other brominated non-condensed ring polyaromatic mixtures are disclosed in U. S. 3,833,674, U. S. 4,717,776, and U. S. 4,740,629.

The particular distribution of the various bromo homologs in the mixture will determine the mixture's average bromine number and its possible effect on the physical properties of articles made thermoplastic formulations containing such mixtures. It is generally desirable to have a high average bromine number since the amount of bromine in the mixture is directly tied to the flame retardant effect per unit weight of the mixture in the formulation. Obtainment of a high average bromine number has heretofore been accomplished by producing mixtures which contain large amounts of the hepta- and octabromo homologs.

While these higher average bromine numbers are beneficial in minimizing the amount of mixture needed to obtain a certain flame retardancy level, the use of large amounts of hepta- and octabromo homologs is not without a significant penalty. It has been found that such homologs contribute to a reduction in an article's impact strength, which reduction is similar to that which occurs when a filler, e.g., talc, $Mg(OH)_2$, or ZnO, is present in the article. To give the mixture a less filler-type nature, the mixture should contain more of the less brominated homologs, e.g., the penta- and hexabromo homologs, and less of the hepta- and octabromo homologs. The lower brominated homologs, i.e., hexabromo and below, will give the mixture a plasticizer-type component which can be balanced against the filler-type component provided by the higher bromo homologs. A major problem with this approach is that the art has not developed a process which produces a mixture which contains significant amounts of the less brominated homologs and which, has, at the same time, a sufficiently high enough average bromine number.

This invention relates to a process for preparing novel mixtures of brominated non-condensed ring polyaromatics, which process comprises: adding bromine to a reactor containing the precursor non-condensed ring polyaromatic, a solvent and a catalytic amount of $AlCl_3$; maintaining the reactor contents, during the bromine addition, at a temperature within the range of 5°C-20°C; terminating the addition of bromine when the number of mols of bromine added is substantially equal to the bromine number sought for the mixture; and after at least substantially all of the added bromine has reacted, recovering the brominated non-condensed ring polyaromatic mixture from the remainder of the reactor contents, the recovered mixture having an average bromine number, based upon GC area percent, within the range of 6.7-7.3 and containing the hexabromo, heptabromo and octabromo homologs of the brominated non-condensed ring polyaromatic in which the amount of the hexabromo homolog is greater than or equal to the sum of the amounts of the heptabromo and the octabromo homologs in the recovered mixture.

The non-condensed ring polyaromatic reactant used in the process of this invention can be represented by the formula

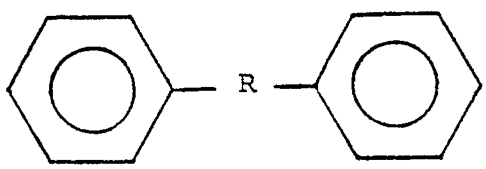

wherein R is an alkylene group of up to 6 carbon atoms, an oxygen atom, a sulfur atom, an oxyalkylene group (-O-R-) of up to 6 carbon atoms, an oxylalkyleneoxy group (-O-R-O-) of up to 6 carbon atoms or a carbon to carbon single bond. Exemplary of these polyaromatic reactants are:

diphenyl oxide,
1,2-diphenyl ethane,
diphenyl,
diphenyl sulfide,
1,5-diphenyl pentane,
benzylphenyl ether, and
1,3-diphenoxyethane.

Based upon present day and anticipated market demand for certain mixtures of this invention, diphenyl oxide and 1,2-diphenyl ethane are the preferred reactants. The diphenyl oxide reactant has been in commercial use for several years and is commercially available. Diphenyl alkanes can be produced by the reaction of benzene and alkylene dihalide in the presence of aluminum trichloride. See CA 97 38651d (Japanese Kokai 82/45114) and CA 46 7084g.

The polyaromatic reactant, catalyst and solvent can be provided to the reactor in any order and in any combination. A preferred manner of addition is to first add a solution of the solvent and the polyaromatic reactant to the reactor and to then add the $AlCl_3$ catalyst. It is preferred that the addition of the materials be at a temperature which is at least near the temperature at which the bromine addition will occur, i.e., 5 °C-20 °C. This is not to say that addition cannot occur at other temperatures. However, when the addition is at elevated temperatures, e.g. 20 °C-35 °C, care must be taken when cooling the reactor contents to the bromine addition temperature to prevent atmospheric moisture from being aspirated into the reactor. The presence of water in the reactor is not desirable as water can deactivate the catalyst.

The solvent used must be one in which the polyaromatic reactant is substantially soluble and in which the produced mixture is substantially insoluble, i.e., at least about 60 weight percent is insoluble. The solvent should also be a liquid during the bromination of the polyaromatic reactant and substantially inert to the process. Generally, halogenated lower alkanes and alkenes are suitable. Exemplary solvents are methylene bromide, methylene chloride, ethylene dibromide, ethylene dichloride, trichloroethylene and mixtures thereof. When using ethylene dichloride, process temperatures which promote transhalogenation of the solvent should be avoided. Preferred solvents are methylene bromide, methylene chloride and mixtures thereof. The most preferred solvent is methylene bromide.

The amount of solvent used is that amount which will at least provide a stirrable reaction mass. Generally, 500-1000 mL, preferably 600 mL - 800 mL of solvent per mole of polyaromatic reactant is used.

The $AlCl_3$ catalyst used in the process of this invention is conventional and is available from commercial sources. Since the catalytic activity of the catalyst is degraded by contact with water, at least near anhydrous conditions should be present before and during the reaction of the polyaromatic reactant and bromine. The catalytic quantities used in the process range from 0.02 mol to 0.15 mol of $AlCl_3$ per mole of polyaromatic reactant. Preferred amounts are within the range of from 0.05 mol to 0.10 mol of $AlCl_3$ per mole of polyaromatic reactant.

The process of this invention is unusual in that it uses $AlCl_3$ as the bromination catalyst. The art recognizes $AlCl_3$ to be a very strong bromination catalyst and it is commonly used to produce perbrominated diphenyl compounds. It would thus be expected that, if $AlCl_3$ is used in producing brominated diphenyl compounds, the bromo homolog distribution would favor the higher brominated homologs. Instead, the process of this invention unexpectedly produces a mixture in which the hexabromo homolog is favored over the more brominated hepta-and octabromo homologs.

Prior to the addition of bromine to the reactor, the polyaromatic reactant, solvent and catalyst in the reactor are at a temperature which is below about 10 °C, and preferably between 0 °C and 10 °C. The temperature can be obtained by charging cold polyaromatic reactant, solvent and/or catalyst to the reactor so as to have the reactor contents at the desired bromine addition temperature. Another method is to charge the three components to the reactor at room temperature and then cool the charge to the desired temperature. During the cooling of the charge, it is prudent, as before noted, to prevent atmospheric moisture from being aspirated into the reactor.

The bromine addition to the cold contents of the reactor should occur soon after the polyaromatic reactant, solvent and catalyst have been charged. The amount of bromine added is that amount which will give the recovered mixture an average bromine number, based upon GC are a percent, which is within the range of 6.7-7.3. Since all of the bromine which is added to the reactor is reacted, and since it takes one mole of bromine, i.e., $Br_2$, per mole of polyaromatic reactant to effect the placement of one bromine atom on the ring, the number of moles of bromine added will substantially equal the average bromine number of the recovered mixture. The term "substantially" is used to describe this equality since it is possible that some of the bromine added will be lost from the reaction due to its entrainment in the stream of HBr being evolved from the reactor contents and/or due to any competing side reactions. Generally, the losses are not great, say 0.50 percent of the bromine added. However, to obtain the desired average bromine number, any losses must be made up by the addition of the excess "make-up" bromine. Thus, in most instances the amount of bromine added will be in slight molar excess of that which equals the average bromine number. For example, to obtain an average bromine number of seven, about 7.04 mols of bromine are usually added. In most instances, 7.0-7.04 mols of bromine will be added.

The bromine can be added to the reactor at any rate which does not cause the reaction mass to overheat and/or does not cause an evolution of by-product HBr which is so great that a safety hazard is

created. From a process efficiency standpoint, it is preferable that the addition rate be as rapid as is possible without realization of significant overheating and/or safety problems. Generally for lab scale processes the bromine addition rate is preferably 0.5 mL/min - 1.0 mL/min. Determining the optimum addition rates for large scale processes will be dependent upon reactor configuration, reaction mass size, reactor cooling equipment available and process economics. This determination is best determined empirically for each different process size and equipment configuration used.

After the bromine addition is complete, the reaction mass is allowed to undergo a ride period until at least substantially all of the bromine has been reacted. There are two signs that can be used to confirm completion of the bromine reaction. The first sign is the loss of a red color in the reaction mass. Use of this sign for determining ride time is most convenient when carrying out the process in a transparent reactor, such as a laboratory glass flask, or a reactor provided with a sight-glass or other direct or indirect viewing means. A second sign is the cessation of HBr evolution from the reactor contents. This sign is more convenient for use with large scale processes.

The ride time is affected by the temperature of the reactor contents after the bromine addition. Shorter ride times are associated with higher reaction mass temperatures and longer ride times are associated with lower temperatures. It is preferred that the temperature of the reactor contents be raised to be within the range of 40° C-60° C during the ride period. Generally, it is not desirable to let the temperature of the reactor content go above about 80° C as adverse side reactions or solvent loss can occur. The maximum temperature is somewhat determined by the particular solvent used. For the preferred solvent, methylene bromide, the maximum temperature during the ride time should not exceed about 60° C.

The temperature of the reactor contents during the ride time can be raised by applying heat thereto. It is preferred, from an operations standpoint, to allow the temperature of the reactor contents to rise naturally after the bromine addition until the temperature rise rate becomes unacceptably slow. Then heat can be applied to bring the reactor contents to the selected maximum temperature.

After the reaction between the bromine and the polyaromatic reactant has at least substantially ceased, the brominated mixture is recovered from the reaction contents. Some of the mixture substituents may be dissolved in the solvent and they need to be at least partially recovered therefrom. One technique that can be used is to contact the reactor contents with a $C_1$ to $C_4$ alkanol. The alkanol acts as a precipitating agent to precipitate at least a portion of the dissolved mixture substituents from the solvent. A preferred alkanol is methanol. The reactor contents and alkanol are brought into contact by adding one to the other. The amount of alkanol used can be within the range of 1-3 volumes of alkanol per volume of the reactor contents. There is no real upper limit to the amount of alkanol that can be used, however, secondary considerations, such as reactor size and process economics, will determine the amount which is sensibly used. The lowest amount of alkanol that is used is that amount which is capable of effecting the recovery sought. The temperature at which the reactor contents and alkanol are contacted is not critical and any convenient temperature can be used. Preferred temperatures are within the range of from ambient temperature to 65° C. The contact can be maintained up to two hours to insure the highest degree of precipitation of the brominated polyaromatic mixture substituents which were in the solvent. Lesser times can be used since most substituents will precipitate out almost immediately. The resultant solid portion of the reactor contents, which comprises the brominated polyaromatic mixture, is recovered by conventional liquid-solid separation methods, e.g., filtration and centrifugation.

The other technique for recovering the brominated polyaromatic mixture involves steam-stripping of the reactor contents to remove the solvent therefrom. The remaining residue is comprised principally of the brominated polyaromatic mixture. Steam stripping has an added advantage in that the steam will deactivate the catalyst.

To reduce the impurities in the recovered brominated polyaromatic mixture, it can be washed with alkanol, caustic, water or all three. After washing, the mixture is then dried to yield a particulate product.

As before noted, the novel brominated polyaromatic mixtures produced by the process of the invention are characterized in that:

(1) they have an average bromine number, based upon GC area percent, within the range of 6.7-7.3;
(2) the hexabromo homolog in the mixtures is present in an amount greater than any other homolog; and
(3) the amount of hexabromo homolog is equal to or greater than the sum of the amounts of the hepta- and octabromo homologs in the mixtures.

Even though the above specifies that only the hexa-, hepta- and octabromo homologs need be present, other bromo homologs will probably be present. For example, when the polyaromatic reactant is diphenyl ethane, pentabromodiphenyl ethane, nonabromodiphenyl ethane and decabromodiphenyl ethane can be present.

The average bromine number is defined as the average number of bromine atoms per molecule of

brominated polyaromatic ethane in the mixture. The average bromine number can be calculated by multiplying the gas chromatographic (GC) area percent or the weight percent of each bromo homolog in the mixture by the number of bromine atoms in that homolog, adding the resulting product and dividing the sum by 100. There will be a slight variation between the average bromine number obtained when using the GC area percent and when using weight percent. This variation can exist because the GC area percent does not always accurately reflect the quantitative relationship between the different bromo homologs in the mixture. The inaccuracy is due to the GC response being different for various of the bromo homologs in the mixture. The variation between GC area percent and weight percent can be resolved by multiplying the GC response factor for each bromo homolog times the GC area percent for that homolog. The product will give the weight percent. For the mixtures of this invention, preferred average bromine numbers, based upon GC area percent, are within the range of 6.7-7.3, with an average bromine number of 6.9-7.1 being most preferred. Most highly preferred are average bromine numbers of 7.0-7.07.

For the purposes of obtaining the GC area percents and the identities of the bromo homologs which form the mixtures produced by the process of this invention, a combination of gas chromatography and mass spectrometry can be used. The mass spectrometer is used to identify each bromo homolog and correlate its identity with the particular peak(s) and retention time(s) shown by the gas chromatogram.

It is recognized that the GC area percent values for each bromo homolog may vary slightly dependent upon the particular gas chromatograph used and upon the analytical conditions used in operating the gas chromatograph. To provide a standard for obtaining the GC area percent values used herein, the following is given:

<u>Gas Chromatography</u>

| | | |
|---|---|---|
| Instrument | - | Hewlitt Packard HP5780-A |
| Column | - | 10 meter, DB-1 methyl silicone megabore, made by J&W Scientific of California |
| Program | - | 200-300°C, at 5°/min + 20 min. hold at 300°C. |
| Injector | - | 285°C, split injector, Hewlitt Packard |
| Detector | - | 325°C FID (flame ionization detector) |
| Sample Size | - | 1 μL (1% soln) + 1 μL methylene bromide plug |

The response factors used in obtaining the weight percent values recited herein are approximates which are based upon empirical observation and experience in quantifying the bromo homologs in various flame retardants.

| Bromo homolog | Response Factor |
|---|---|
| DPO/DPE-$BR_4$ | 0.85 |
| DPO/DPE-$BR_5$ | 0.85 |
| DPO/DPE-$BR_6$ | 0.9 |
| DPO/DPE-$BR_7$ | 0.9 |
| DPO/DPE-$BR_8$ | 1.0 |
| DPO/DPE-$BR_9$ | 1.1 |
| DPO/DPE-$BR_{10}$ | 1.1 |

More exact response factors can be obtained by conventionally comparing the GC area percents of a known amount of each brominated homolog against one another.

On the basis of GC area percent, a typical bromo homolog distribution for brominated diphenyl ethane mixtures of this invention is, 0-2 GC area percent pentabromodiphenyl ethane, 40-55 GC area percent hexabromodiphenyl ethane, 12-25 GC area percent heptabromodiphenyl ethane, 15-25 GC area percent octabromodiphenyl ethane, 10-15 GC area percent nonabromodiphenyl ethane, and 0-5 GC area percent decabromodiphenyl ethane. The mixture can also contain some impurities, usually less than 1.5 GC area percent. On the basis of weight percent, this typical mixture will contain 0-2 weight percent pentabromodiphenyl ethane, 34-47 weight percent hexabromodiphenyl ethane, 11-23 weight percent heptabromodiphenyl ethane, 15-25 weight percent octabromodiphenyl ethane, 11-17 weight percent nonabromodiphenyl ethane, and 0-6 weight percent decabromodiphenyl ethane.

When the mixtures are derived from diphenyl oxide a bromo homolog distribution similar to that for diphenyl ethane can be obtained by use of the process of this invention.

The brominated polyaromatic mixtures produced by the process of this invention, especially the brominated diphenylethane mixtures, and most especially those mixtures having an average bromine number of 6.8-7.2, are useful as flame retardants in ABS, i.e., acrylonitrilebutadiene-styrene, based formulations. The amount used to achieve the desired flame retarded effect is generally from 12 weight percent to 25 weight percent, based upon the total weight of the formulation. It is preferred that the formulation also contain any of the well known flame retardant synergists which are commonly used with halogen containing flame retardants. Such synergists enhance the flame retardant qualities of the brominated polyaromatics in the mixture and thus enable the use of lesser amounts of the mixture to obtain the desired flame retardant effect. Examples of such synergists are $Sb_2O_3$, $Sb_2O_4$, $Sb_2O_5$, zinc oxide, zinc borate, various inorganic bismuth compounds, and organic compounds such as tris-2-chloroethylphosphate and tris-2,3-dibromopropylphosphate. The most preferred synergist is $Sb_2O_3$.

The flame retardant synergist will generally be used in an amount, based upon the total weight of the ABS based formulation, which is within the range of from 2 weight percent to 6 weight percent. When a flame retardant synergist is used, the amount of brominated polyaromatic mixture used is preferably within the range of from 10 weight percent to 20 weight percent.

The ABS resin can be any of those which are denominated by the art as high impact, medium impact, low impact or heat resistant. The ABS resin can be comprised of any suitable proportion of acrylonitrile, rubber and styrene. The resin can also be any of those produced by the well known emulsion, suspension or batch processes. Even further, the resin may have units other than acrylonitrile, butadiene and styrene. For example, methylmethacrylate can be copolymerized therewith. Also, other polymers may be used to modify the ABS resin, such other polymers including modified styrene resins, such as rubber modified polystyrenes, and the styrene containing copolymers, such as the styrene-acrylonitrile copolymers, styrene-butadiene copolymers, styrene-acrylonitrile-$\alpha$-alkylstyrene copolymers, poly-$\alpha$-methylstyrene, copolymers of ethylvinylbenzene and divinylbenzene, and the like. The preferred resin is unmodified acrylonitrilebutadiene-styrene. For a further discussion of suitable ABS resins, see Kirk-Othmer Encyclopedia of Chemical Technology, 3rd edition, John Wiley & Sons, Vol. 1, pages 442-456, and Encyclopedia of Polymer Science and Technology, John Wiley & Sons, Vol. 1, pages 436-444.

The ABS resin constituent used in the formulation will comprise from 70 to 90 weight percent of the formulation and preferably from 75 to 90 weight percent.

The constituents of the ABS based formulation can be blended one with the other in any order and by

way of any conventional technique. A Branbury mixer or twin screw extruder can be used.

The ABS based formulation can also contain conventional additives, for example, plasticizers, pigments, antioxidants, fillers, e.g., talc, glass, UV stabilizers, processing aids and the like.

Conventional article forming techniques can be used to form articles from the above described ABS based formulations. For example injection molding, compression molding, and extrusion molding are all suitable.

The following Examples illustrate some of the features of the inventions hereinabove disclosed and are not to be taken as limiting such inventions.

## EXAMPLES

The following equipment was used in Examples I-III. A 500 mL, 5-necked reaction flask was fitted with a Friedrich's condenser modified for use as a dry-ice/IPA cold-finger condenser. The reactor overhead led from the condenser exit to an oil bubbler charged with inert fluorocarbon oil, a safety trap and a tared caustic trap. The dip-leg to the caustic trap was positioned just below the liquid surface; the trap itself was placed on a balance to measure HBr evolution quantitatively with reaction time. Alternatively, the trap could be stirred magnetically if HBr weight was not a concern. A nitrogen line was tied into the overhead, downstream from the condenser. A 3-way Teflon stopcock allowed a $N_2$ purge to maintain positive pressure when HBr flow became weak, thus preventing caustic suck-back. To clear most of the residual HBr from the system, the nitrogen purge could be transferred to the 3-way stopcock on the side-arm of the empty bromine addition funnel. With the stopcock open, the addition funnel, reactor and condenser vapor spaces could be flushed at a controlled rate. The addition funnel itself was fitted with a 2 mm, metered Teflon stopcock. A thermocouple-thermowell was placed in the fourth reactor neck; the fifth neck was used for catalyst addition.

### EXAMPLE I

The reactor was charged with 18.2 g, (0.10 M) diphenyl ethane, hereinafter DPE, and methylene bromide (50 mL), the solution was chilled to 6° C and $AlCl_3$ (0.87g) was added. Bromine (112.0g, 0.70 M) was added over 1.0 hour at 6-8° C. Solids deposition was observed at 61% bromine addition. The yellow mixture was heated over 0.3 hour to 18° C, yielding a tan slurry. Heating continued over 1.2 hours to 54° C maximum.

Methanol (100 mL) was added dropwise at 65° C (the first few drops turned the slurry white) over 0.4 hour. The thick, white slurry was cooled, filtered, washed with methanol and air-dried to give a white powder (69.3g, 94.3% yield). The bromo homolog distribution and average bromine number are given in Table I. The filtrate/wash residue was obtained as a gummy solid. This was leached twice with methanol and air-dried to give a tan powder (5.0g).

### EXAMPLE II

(Comparative Example)

The reactor was charged with DPE (18.2 g, 0.10 M) and methylene bromine (50 mL). The reactor was chilled to 8° C and $FeBr_3$ (1.04 g) was added. Bromine (112.0 g, 0.70 M) was added over 0.8 hour at 8-15° C; solids formation occurred with 81% bromine charged. The slurry was then heated to 89° C over 1.8 hours, when additional bromine (3.1 g) was added to compensate for a small overhead loss past the condenser cold-finger. The mixture was heated an additional 1.0 hour at 87-95° C, giving 97.1% HBr theory in the caustic trap.

The mixture was cooled to 70° C and methanol (100 mL) was added over 0.3 hour at slow reflux. Solvent color persisted, but small particles of white solids were observed on the reactor walls. After cooling, the reaction mass was filtered and the filter cake slurry-washed several times until the filtrate was colorless. Drying gave a white powder (72.2 g, 98.2% yield). The bromine content and the bromo homolog distribution are given in Table I.

### EXAMPLE III

A 3-liter resin kettle was charged with DPE (145.8 g, 0.80 M) and methylene bromide (400 mL). The kettle was sealed and the contents stirred while cooling the solution in an ice-bath. At 6° C, $AlCl_3$ (7.62 g)

was charged to the reactor, while bromine (894.9 g, 5.60 M) was transferred to the 500 mL addition funnel. The bromine was added over 1.3 hours at 6-9 °C; solids formed with only 38% bromine charged. An additional increment of bromine (3.1 g, 0.019 M) was added to compensate for overhead losses past the cold-finger. The thick slurry was heated, over 2.0 hours, to 50 °C and gave a dark-tan slurry. Methanol (800 mL) was added, over 0.5 hour, at slow reflux to yield a cream-colored mixture containing lumpy solids instead of fine crystals. The cooled mixture was filtered, washed and the solids air-dried. After grinding with a mortar and pestle, the product was again air-dried to give an off-white powder (548.0 g, 93.2% yield).

EXAMPLE IV

The resin kettle used in Example III was charged with DPE (150.8 g, 0.837 M) and methylene bromide (415 mL) and the solution was stirred. The curved adapters leading from the cold-finger and the addition funnel were modified with the addition of 3-inch extensions to help contain bromine vapor in the reactor. After cooling to 5 °C, $AlCl_3$ (8.0 g) was charged and bromine (939.0 g, 5.876 M, 7.10 equiv.) was transferred to the addition funnel. The bromine was added over 2.1 hours at 7-9 °C; solids formation began with 41% charged. The yellow-tan slurry was heated, over 2.2 hours, to 57 °C; methanol (830 mL) was added, over 1.3 hours, at 57-69 °C. Large, tan particles separated with 50% methanol charged.

The product was worked up as before, to give (after grinding) a dark-tan powder (578.3 g, 94.2% yield). This material was charged to a 3-liter reactor with C.P. acetone (1-liter); the slurry was stirred at reflux for 0.5 hours. After cooling, the solids were filtered, washed with cold acetone (200 mL) and air-dried to give an off white powder (539.4 g, 87.9% yield) which was comparable to the product of Example III. The dark-yellow acetone filtrate was concentrated to 200 mL and cooled. The resulting precipitate was filtered and dried to give a white powder (21.0 g, 3.4% yield) which was blended with the main product. Total solvent removal gave a yellow, gummy material (13.4 g) which was not mixed with the product.

EXAMPLE V

The products of both Examples III and IV were blended manually. After blending, the particle size was unacceptable (58.5 average). The blend was then air-milled to give a much smaller particle (5.4 average) which was suitable for mixing with ABS resin. The average bromine number and the bromo homolog distribution for this product blend is given in Table I.

TABLE I

| | Product from Example No. | | |
|---|---|---|---|
| | I | II | V |
| Constituent | GC area % | GC area % | GC area % |
| DPE-$BR_4$ | --- | --- | 0.09 |
| DPE-$BR_5$ | 1.10 | 0.17 | 2.76 |
| DPE-$BR_6$ | 48.29 | 36.70 | 41.68 |
| DPE-$BR_7$ | 15.52 | 27.95 | 17.83 |
| DPE-$BR_8$ | 17.15 | 22.53 | 20.55 |
| DPE-$BR_9$ | 13.92 | 10.42 | 12.70 |
| DPE-$BR_{10}$ | 2.78 | 2.24 | 2.52 |
| Light ends | 1.24 | --- | 1.97 |
| Melting Point Range | 163-210°C | 150-210°C | 150-205°C |
| Average Br Number * | 7.03 | 7.13 | 7.06 |

* Average Br number for Examples I and V obtained by normalizing GC area percent to not include Light Ends.

As can be seen from the Table, the mixtures of this invention principally contain the hexa-, hepta- and octabromo homologs, with the predominate amount belonging to the hexabromo homolog. Despite this distribution, the average bromine number, based upon GC area percent, is 7.02 and 7.06 for Examples I and V, respectively.

EXAMPLE VI

Three ABS-based formulations were prepared using a Brabender mixer. Two of the formulations contained 4 weight percent $Sb_2O_3$, 77.3 weight percent Cycolac T-1000, an ABS resin sold by Borg-Warner Corporation (now General Electric Company), and 18.7 weight percent of the brominated diphenyl ethane mixture from Example I or Example II. The third formulation contained 100 weight percent Cycolac T-1000. Each formulation was compression molded at a temperature of 177° C and at a molding pressure of 1400-1800 gram-meter torque to form test specimens which are identified in Table II.

TABLE II

| Test Specimen | Formulation Composition | UL-94 Rating | ASTM D 256 Izod Impact 1/8" notch Ft.lb/in. notch |
|---|---|---|---|
| 1 | Cycolac T-1000<br>Example I mixture<br>$Sb_2O_3$ | V-O | 1.5 |
| 2 | Cycolac T-1000<br>Example II mixture<br>$Sb_2O_3$ | V-O | 0.90 |
| 3 | Cycolac T-1000 | burn | 4.6 |

As can be seen from Table II, a brominated diphenyl ethane mixture of this invention (Specimen No. 1) gave a UL-94 V-O rating with less of an adverse affect on the specimen's Izod Impact strength than was realized when using a diphenyl ethane mixture not of this invention.

## Claims

1. A process of preparing a mixture of brominated non-condensed ring polyaromatics, which process comprises:
   (a) adding bromine to a reactor initially containing a solvent, a catalytic amount of aluminum chloride, and a a non-condensed ring polyaromatic of the formula

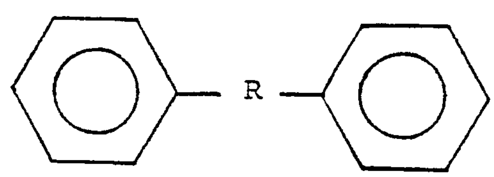

   wherein R is an alkylene group of up to 6 carbon atoms, an oxygen atom, a sulfur atom, an oxyalkylene group of up to 6 carbon atoms, an oxyalkyleneoxy group of up to 6 carbon atoms or a carbon to carbon single bond;
   (b) maintaining the reactor contents during the bromine addition at a temperature of from 5 °C to 20 °C;
   (c) terminating the addition of bromine when the number of mols of bromine added is substantially equal to the bromine number sought for the mixture; and
   (d) after at least substantially all of the bromine has reacted, recovering the brominated non-condensed ring polyaromatic mixture from the remainder of the reactor contents, the recovered mixture having an average bromine number, based upon GC area percent, within the range of 6.7-7.3 and containing hexabromo, heptabromo and octabromo homologs of the non-condensed ring polyaromatic, in which the amount of the hexabromo homolog is greater than or equal to the sum of the amounts of the heptabromo and the octabromo homologs in the recovered mixture.

2. The process of Claim 1 wherein the non-condensed ring polyaromatic is diphenyl oxide or 1,2-diphenyl ethane.

3. The process of Claim 1 or 2 wherein the solvent is methylene bromide, methylene chloride or a mixture thereof; there are 500-1000 mLs of solvent per mole of non-condensed ring polyaromatic; the catalytic amount of aluminum chloride is within the range of 0.02-0.15 mol of aluminum chloride per mole of non-condensed ring polyaromatic; and the amount of bromine added to the reactor is 7.00-7.04 mols of bromine per mole of non-condensed ring polyaromatic.

4. The process of Claim 3 wherein the solvent is methylene bromide.

5. The process of any of the preceding claims wherein the non-condensed ring polyaromatic is 1,2-diphenyl ethane and wherein the recovered mixture contains 0-2 weight percent pentabromodiphenyl ethane, 34-47 weight percent hexabromodiphenyl ethane, 11-23 weight percent heptabromodiphenyl ethane, 15-25 weight percent octabromodiphenyl ethane, 11-17 weight percent nonabromodiphenyl ethane, 0-6 weight percent decabromodiphenyl ethane.

6. A mixture of brominated diphenyl ethanes, said mixture having an average bromine number, based upon GC area percent, which is in the range of 6.8-7.2 and containing hexabromodiphenyl ethane, heptabromodiphenyl ethane and octabromodiphenyl ethane, in which the hexabromodiphenyl ethane is present in an amount which is greater than or equal to the sum of the amount of heptabromodiphenyl ethane and the amount of octabromodiphenyl ethane in the mixture.

7. The mixture of Claim 6 wherein said mixture contains 0-2 weight percent pentabromodiphenyl ethane, 34-47 weight percent hexabromodiphenyl ethane, 11-23 weight percent heptabromodiphenyl ethane, 15-25 weight percent octabromodiphenyl ethane, 11-17 weight percent nonabromodiphenyl ethane and 0-6 weight percent decabromodiphenyl ethane.

8. An ABS based formulation containing, in a flame retardant amount, the mixture of Claim 6 or 7 and a predominate amount of an ABS resin.

9. The ABS based formulation of Claim 8 wherein the formulation additionally contains a flame retardant synergist.

10. An article formed from the ABS based formulation of Claim 8 or 9.